(19) Europäisches Patentamt

European Patent Office

Office européen des brevets

(11) **EP 0 636 606 B1**

(12) **EUROPÄISCHE PATENTSCHRIFT**

(45) Veröffentlichungstag und Bekanntmachung des Hinweises auf die Patenterteilung:
**29.05.1996 Patentblatt 1996/22**

(51) Int. Cl.$^6$: **C07C 303/38**, C07C 311/03

(21) Anmeldenummer: **94111136.1**

(22) Anmeldetag: **18.07.1994**

(54) **Verfahren zur Herstellung flüssiger Aminaddukte fluorgruppenhaltiger Amide**

Process for the preparation of liquid amine-adducts of amides containing fluorinated groups

Procédé de préparation d'adduits liquides d'une amine avec une amide contenant des groupes fluorés

(84) Benannte Vertragsstaaten:
**BE DE ES FR GB IT NL**

(30) Priorität: **29.07.1993 DE 4325485**

(43) Veröffentlichungstag der Anmeldung:
**01.02.1995 Patentblatt 1995/05**

(73) Patentinhaber: **BAYER AG**
**51368 Leverkusen (DE)**

(72) Erfinder:
• **Schneider, Wolfgang, Dipl.-Ing.**
**D-51375 Leverkusen (DE)**
• **Stachulla, Karlheinz**
**D-51375 Leverkusen (DE)**
• **Pohmer, Klaus, Dr.**
**D-51061 Köln (DE)**
• **Weber, Rainer, Dr.**
**D-51519 Odenthal (DE)**
• **Schlak, Ottfried, Dr.**
**D-51061 Köln (DE)**
• **Moretto, Hans-Heinrich, Dr.**
**D-51375 Leverkusen (DE)**

(56) Entgegenhaltungen:
**EP-A- 0 021 003       EP-A- 0 439 687**
**US-A- 4 296 034**

• **CHEMICAL ABSTRACTS, vol. 114, no. 1, 7.**
**Januar 1991, Columbus, Ohio, US; abstract no.**
**5775p, PODOL'SKII, A.V. ET AL. 'Preparation of**
**ammonium salts of**
**perfluoroalkanesulfonamides'**
• **CHEMICAL ABSTRACTS, vol. 114, no. 1, 7.**
**Januar 1991, Columbus, Ohio, US; abstract no.**
**5775p, PODOL'SKII, A.V. ET AL. 'Preparation of**
**ammonium salts of**
**perfluoroalkanesulfonamides' & ZH.OBSHCH.**
**KHIM. Bd. 60, Nr.6, 1990 Seiten 1387 - 1390**

**Beschreibung**

Die Erfindung betrifft ein verbessertes Verfahren zur Herstellung von flüssigen Aminaddukten fluorgruppenhaltiger Amide, bei dem die Verbindungen in zwei Verfahrensschritten ohne Synthese von Zwischenprodukten lösungsmittelfrei synthetisiert werden.

Flüssige Aminaddukte fluorgruppenhaltiger Amide finden aufgrund ihrer hohen Grenzflächenaktivität vielfältige Verwendung in der Technik. Typische Anwendungen sind die Verwendung als Emulgatoren in der Polymerisation, als Verlaufs- und Netzmittel in der Fotografietechnik bzw. Filmherstellung, als Verlaufs- und Netzmittel in wasserverdünnbaren Lacken (EP-B 0 021 003), oder als Spreitmittel in Feuerlöschschäumen.

Beispiele für solche Verbindungen sind:

$$C_8F_{17}SO_2NH_2 \cdot N(CH_3)_3 \text{ und}$$

$$C_4F_9SO_2NH_2 \cdot N(C_2H_5)_3$$

Die Herstellung von Aminaddukten erfolgt gemäß EP-B 0 021 003 durch UmSetzung von Perfluoralkylsulfonamiden mit tertiären einen gemäß Gleichung (1):

$$C_nF_{2n+1}SO_2NHR_H + N(R^1R^2R^3) \rightarrow C_nF_{2n+1}SO_2NHR_H \cdot N(R^1R^2R^3) \tag{1}$$

wobei

n    eine ganze Zahl zwischen 4 und 20 und

$R^1$, $R^2$, $R^3$  unabhängig voneinander ein Wasserstoffatom, Alkyl-, Hydroxyalkyl- oder Alkoxyalkylrest mit 1 - 4 Kohlenstoffatomen und

$R_H$    ein Wasserstoffatom, ein Alkyl-, Hydroxyalkyl- oder Alkoxyalkylrest mit 1 - 4 Kohlenstoffatomen sind.

Das für diese Reaktion benötigte Perfluoralkylsulfonamid erhält man z.B. gemäß T. Granstad, R.N. Haszeldine, J. Chem. Soc. (1958) 2640-45 durch Umsetzung von Perfluoralkylsulfonylfluorid mit Ammoniak unter Verwendung von organischen Lösungsmitteln, z.B. in Diisopropylether, gemäß Gleichung (2):

$$C_nF_{2n+1}SO_2F + 2\,NH_3 \xrightarrow{\text{Lösungsmittel}} C_nF_{2n+1}SO_2NH_2 + NH_4F \tag{2}$$

Das Lösungsmittel hat bei dieser Umsetzung verschiedene Funktionen. Es dient z.B. als Extraktionsmittel zur Abtrennung des Perfluoralkylsulfonamids aus Ammoniumfluorid. In einem geeigneten Lösungsmittel löst sich das Perfluoralkylsulfonamid, nicht aber das gleichzeitig entstandene Ammoniumfluorid. Letzteres kann z.B. durch Filtration abgetrennt werden. Weiterhin kann das Lösungsmittel als Trägerflüssigkeit für das bei entsprechender Reaktionstemperatur gasförmige Ammoniak dienen. Da Perfluoralkylsulfonamide bei Raumtemperatur Feststoffe sind, wird durch Zusatz von Lösungsmittel eine Reaktionsführung in flüssiger Phase möglich, was erhebliche verfahrenstechnische Vorteile bringt, wie z.B. bessere Durchmischung des Reaktionsgemisches und damit bessere Temperierbarkeit und gegebenenfalls schnelleren Reaktionsverlauf. Ist das Lösungsmittel derart gewählt, daß es mit Wasser nicht mischbar ist, kann eine Reinigung der organischen Phase durch Wäsche erfolgen.

Zur Isolierung des Sulfonamids kann der lösungsmittelhaltige Ansatz mit wäßriger Schwefel- oder Salzsäure gereinigt und die wäßrige, salzhaltige Phase abgetrennt werden. Die lösungsmittelhaltige Phase muß mehrfach mit demineralisiertem Wasser nachgewaschen werden. Danach wird das Lösungsmittel destillativ abgetrennt. Man erhält ein sehr reines Perfluoralkylsulfonamid.

Nachteile beim Einsatz von Lösungsmitteln zur Herstellung von Perfluoralkylsulfonamiden sind z.B. die deutlich reduzierte Raum-Zeit-Ausbeute und das Gefahrenpotential, das häufig von diesen Lösungsmitteln ausgeht, insbesondere die mögliche Peroxidbildung in Ethern. Weiterhin kommt es durch die Produktwäschen zu einem erheblichen Anfall an belastetem Abwasser, außerdem ist eine Aufarbeitung oder Vernichtung der Lösungsmittel notwendig.

Die Aufgabe bestand darin, ein sicheres, einfaches und kostengünstiges Verfahren zur Herstellung von flüssigen Aminaddukten fluorgruppenhaltiger Amide zur Verfügung zu stellen, das die obengenannten Nachteile nicht aufweist.

Überraschenderweise wurde nun gefunden, daß man direkt, ohne den Umweg über ein Zwischenprodukt, zu flüssigen Aminaddukten fluorgruppenhaltiger Amide kommt, wenn man fluorgruppenhaltige Sulfonsäurehalogenide stöchiometrisch mit einem tertiären Amin und Ammoniak oder einem primären Amin umsetzt. Nach anschließender Filtration bei einem Druck von 1 bis 6 bar und einer Temperatur von 20 bis 120°C erhält man ein hochreines Produkt mit einem Halogenidgehalt kleiner 0,1 % gemäß Gleichung (3):

$$R_F\text{-}(CH_2)_n\text{-}SO_2\text{-}X + 2\ NH_2R_H + N\ (R^1R^2R^3) \rightarrow R_F\text{-}(CH_2)_n\text{-}SO_2\text{-}NHR_H \cdot N(R^1R^2R^3) + R_HNH_3X \qquad (3),$$

wobei

$R_F$        ein Perfluoralkylrest mit 3 bis 10 Kohlenstoffatomen oder ein Fluoralkylrest mit 3 bis 10 Kohlenstoffatomen ist,

$n$        eine ganze Zahl zwischen 0 und 6 darstellt,

$X$        ein Halogenatom ist,

$R_H$        ein Wasserstoffatom oder einen linearen Alkylrest mit 1 bis 4 Kohlenstoffatomen darstellt,

$R^1, R^2$ und $R^3$        unabhängig voneinander einen linearen Alkylrest mit 1 bis 4 Kohlenstoffatomen darstellt.

Gegenstand der Erfindung ist ein Verfahren zur Herstellung flüssiger Aminaddukte fluorgruppenhaltiger Amide der allgemeinen Formel (I)

$$R_F\text{-}(CH_2)_n\text{-}SO_2\text{-}NHR_H \cdot N(R^1R^2R^3) \qquad (I),$$

wobei

$R_F$        ein Perfluoralkylrest mit 3 bis 10 Kohlenstoffatomen oder ein Fluoralkylrest mit 3 bis 10 Kohlenstoffatomen ist,

$n$        eine ganze Zahl zwischen 0 und 6 darstellt,

$R_H$        ein Wasserstoffatom oder einen linearen Alkylrest mit 1 bis 4 Kohlenstoffatomen darstellt,

$R^1, R^2$ und $R^3$        unabhängig voneinander einen linearen Alkylrest mit 1 bis 4 Kohlenstoffatomen darstellen,

dadurch gekennzeichnet, daß ein fluorgruppenhaltiges Sulfonsäurehalogenid der allgemeinen Formel (II)

$$R_F(CH_2)_n\text{-}SO_2\text{-}X \qquad (II),$$

wobei

$R_F$ und $n$     dieselbe Bedeutung wie oben haben und
$X$            für ein Halogenatom steht,

ein tertiäres Amin $N(R^1R^2R^3)$, wobei $R^1$, $R^2$ und $R^3$ dieselbe Bedeutung wie oben haben, und Ammoniak oder ein primäres Amin $H_2NR_H$, wobei $R_H$ dieselbe Bedeutung wie oben hat, ohne Lösungsmittel gleichzeitig miteinander zur Reaktion gebracht werden, und das erhaltene Produkt durch Filtration bei einem Druck von 1 bis 6 bar und einer Temperatur von 20 bis 120°C von ausgefallenen Ammoniumhalogeniden befreit wird.

Besonders bevorzugt ist ein solches Verfahren zur Herstellung flüssiger Aminaddukte fluorgruppenhaltiger Amide, bei dem $R_F$ für einen Perfluoralkylrest mit 4 bis 8 Kohlenstoffatomen steht.

Vorzugsweise werden Sulfonsäurehalogenide mit $n = 0$ eingesetzt.

Verfahren zur Herstellung flüssiger Aminaddukte fluorgruppenhaltiger Amide, bei denen vor der Filtration ein Filterhilfsmittel und/oder Kristallisationshilfsmittel zugesetzt werden, sind bevorzugt.

Besonders bevorzugt sind solche Verfahren zur Herstellung flüssiger Aminaddukte fluorgruppenhaltiger Amide, bei denen Kieselsäurederivate und/oder Zellulosepulver zugesetzt werden.

Verfahren zur Herstellung flüssiger Aminaddukte, bei denen vor der Filtration Wasser in einer Menge von 1 bis 4 Gew.-%, bezogen auf die Masse des Produktes, zugesetzt wird, sind bevorzugt.

Besonders bevorzugt sind solche Verfahren, bei denen Wasser in einer Menge von 1,5 bis 2,5 Gew.-%, bezogen auf die Masse des Produktes, zugegeben wird.

Das erfindungsgemäße Verfahren wird ohne Lösungsmittel durchgeführt.

Das erfindungsgemäße Verfahren wird im folgenden näher erläutert.

## Beispiele

In einem Reaktionsbehälter wird vorzugsweise bei einer Temperatur von -40°C bis +10°C fluorgruppenhaltiges Sulfonsäurehalogenid und tertiäres Amin vorgelegt und Ammoniak bzw. primäres Amin unter starkem Rühren zugesetzt oder tertiäres Amin und Ammoniak bzw. primäres Amin vorgelegt und fluorgruppenhaltiges Sulfonsäureamid zugesetzt. Die Reaktion ist exotherm und sollte durch Kühlung und langsame Dosierung des Reaktanten bei max. +10°C gehalten werden. Es entsteht eine beige-gefärbte Flüssigkeit aus Aminaddukt des fluorgruppenhaltigen Amids und Ammoniumfluorids.

Das entstehende Ammoniumfluorid ist im Reaktionsgemisch unlöslich. Es fällt in sehr feinkörniger Form an. Durch Filtration wird das Ammoniumfluorid entfernt.

Die Vorteile des lösungsmittelfreien Verfahrens liegen in der größeren Verfahrenssicherheit, in einer verbesserten Raum-Zeit-Ausbeute, in einer verminderten Abwasserbelastung, in einer erheblichen Reduzierung des manuellen Arbeitsaufwandes und in der Verbesserung der Qualität des Endproduktes durch geringe Temperaturbelastung. Die Lösungsmittel-Aufarbeitung bzw. -Vernichtung entfällt.

## Beispiel 1

In einer Dreihalskolbenrührapparatur werden 200 g (0,30 mol) Perfluoroctylsulfonylfluorid mit einer Reinheit von >98 % und 40,4 g (0,4 mol) Triethylamin mit einer Reinheit von >99 % vorgelegt. Unter intensivem Rühren werden 18 g (1,06 mol) gasförmiges Ammoniak bei einer Temperatur von -5°C bis +10°C eingeleitet. Das erhaltene Reaktionsgemisch wird mit 5 % Kieselgur versetzt und in einer Drucknutsche mit Filter (Seitz K 300®) bei 50°C und 3 bar filtriert.

| Ausbeute | 133,5 g (entsprechend 57 % der Theorie) |
|---|---|
| Fluoridgehalt | 0,01 % |
| Oberflächenspannung | 24,3 mN/m (100 mg/l Wasser bei 20°C) |

## Beispiel 2

In einer Dreihalskolbenrührapparatur werden 200 g (0,39 mol) Perfluoroctylsulfonylfluorid mit einer Reinheit von >98 % und 40,4 g (0,4 mol) Triethylamin mit einer Reinheit von >99 % vorgelegt. Unter intensivem Rühren werden 18 g (1,06 mol) gasförmiges Ammoniak bei einer Temperatur von -5°C bis +10°C eingeleitet. Das erhaltene Reaktionsgemisch wird mit 5 % Cellulosepulver versetzt und in einer Drucknutsche mit Filter (Seitz K 300®) bei 50°C und 3 bar filtriert.

| Ausbeute | 148 g (entsprechend 63 % der Theorie) |
|---|---|
| Fluoridgehalt | 0,02 % |
| Oberflächenspannung | 24,5 mN/m (100 mg/l Wasser bei 20°C) |

## Beispiel 3

In einer Dreihalskolbenrührapparatur werden 1340 g (2,6 mol) Perfluoroctylsulfonylfluorid mit einer Reinheit von >98 % und 70 g (0,22 mol) Perfluorbutylsulfonylfluorid mit einer Reinheit von 95 % und 300 g (2,97 mol) Triethylamin mit einer Reinheit von >99 % vorgelegt. Unter intensivem Rühren werden 130 g (7,64 mol) gasförmiges Ammoniak bei einer Temperatur von -10°C bis +10°C eingeleitet. Das erhaltene Reaktionsgemisch wird mit 36 g (2 mol) Wasser versetzt und nach einer Reifezeit von 18 Stunden in einer Drucknutsche mit Filter (Seits K 300®) bei 20°C und 3 bar filtriert.

| Ausbeute | 1471 g (entsprechend 86 % der Theorie) |
|---|---|
| Fluoridgehalt | 0,08 % |
| Oberflächenspannung | 25,5 mN/m (100 mg/l Wasser bei 20°C) |

### Beispiel 4

In einer Dreihalskolbenrührapparatur werden 1359 g (4,3 mol) Perfluorbutylsulfonylfluorid mit einer Reinheit von 95 % und 466 g (4,6 mol) Triethylamin mit einer Reinheit von >99 % vorgelegt. Unter intensivem Rühren werden 202 g (11,9 mol) gasförmiges Ammoniak bei einer Temperatur von -10°C bis +10°C eingeleitet. Das erhaltene Reaktionsgemisch wird mit 40 g (2,2 mol) Wasser versetzt und nach einer Rührzeit von 24 bis 36 Stunden in einer Drucknutsche mit Filter (Seitz K 300®) bei 20°C und 3 bar filtriert.

| Ausbeute | 1520 g (entsprechend 78 % der Theorie) |
|---|---|
| Fluoridgehalt | 0,08 % |
| Oberflächenspannung | 57,8 mN/m (100 mg/l Wasser bei 20°C) |

### Beispiel 5

In einer Dreihalskolbenrührapparatur werden 1000 g (2,49 mol) Perfluorhexylsulfonylfluorid mit einer Reinheit von >98 % und 257,5 g (2,55 mol) Triethylamin mit einer Reinheit von >99 % vorgelegt. Unter intensivem Rühren werden 130 g (7,64 mol) gasförmiges Ammoniak bei einer Temperatur von -5°C bis +10°C eingeleitet. Das erhaltene Reaktionsgemisch wird mit 36 g (2 mol) Wasser versetzt und nach 48 Stunden Reifung in einer Drucknutsche mit Filter (Seitz K 300®) bei 20°C und 3 bar filtriert.

| Ausbeute | 1144 g (entsprechend 93 % der Theorie) |
|---|---|
| Fluoridgehalt | 0,09 % |
| Oberflächenspannung | 39,6 mN/m (100 mg/l Wasser bei 20°C) |

### Vergleichsbeispiel

400 g $C_8F_{17}SO_2F$ in 800 ml Diisopropylether werden vorgelegt und auf 5°C unter Rühren abgekühlt. Bei einem Unterdruck von ca. 500 mbar werden während ca. 1,3 h 55 g $NH_3$ Gas so eingeleitet, daß die Sumpftemperatur nicht über 16°C steigt. Anschließend wird ca. 1 Stunde bei Raumtemperatur nachgerührt. Unter Kühlung und Rühren werden 850 ml 11%ige HCl tropfenweise zugegeben. Nach der HCl-Zugabe wird kürz auf 45°C aufgeheizt, dann abgekühlt und die Phasen getrennt. Die organische Phase wird mit einer Lösung aus 17,5 g Eisen(II)-sulfat und 55 g konz. HCl in 400 ml Wasser gewaschen. Anschließend werden die Phasen getrennt. Der Ether wird aus der organischen Phase abdestilliert. Der verbleibende Rest wird getrocknet. Bei 110 bis 120°C werden unter Rühren 111 ml Triethylamin während ca. 45 Minuten zugesetzt, wobei anfangs starke Nebelbildung auftritt. Es wird 10 Minuten bei 120 bis 130°C nachgerührt und auf 40°C abgekühlt. Die Ausbeute beträgt 422,9 g.

### Patentansprüche

1. Verfahren zur Herstellung flüssiger Aminaddukte fluorgruppenhaltiger Amide der allgemeinen Formel (I)

$$R_F\text{-}(CH_2)_n\text{-}SO_2\text{-}NHR_H \cdot N(R^1R^2R^3) \qquad (I),$$

wobei

$R_F$      ein Perfluoralkylrest mit 3 bis 10 Kohlenstoffatomen oder ein Fluoralkylrest mit 3 bis 10 Kohlenstoffatomen ist,

n      eine ganze Zahl zwischen 0 und 6 darstellt,

$R_H$      ein Wasserstoffatom oder einen linearen Alkylrest mit 1 bis 4 Kohlenstoffatomen darstellt,

$R^1, R^2$ und $R^3$      unabhängig voneinander einen linearen Alkylrest mit 1 bis 4 Kohlenstoffatomen darstellt,

dadurch gekennzeichnet, daß ein fluorgruppenhaltiges Sulfonsäurehalogenid der allgemeinen Formel (II)

$$R_F(CH_2)_n\text{-}SO_2\text{-}X \qquad\qquad (II),$$

wobei

$R_F$ und n dieselbe Bedeutung wie oben haben und

X für ein Halogenatom steht,

ein tertiäres Amin $N(R^1R^2R^3)$ und Ammoniak oder ein primäres Amin $H_2NR_H$, wobei $R^1$, $R^2$, $R^3$ und $R_H$ dieselbe Bedeutung wie oben haben, ohne Lösungsmittel gleichzeitig miteinander zur Reaktion gebracht werden, und das erhaltene Produkt durch Filtration bei einem Druck von 1 bis 6 bar und einer Temperatur von 20 bis 120°C von ausgefallenen Ammoniumhalogeniden befreit wird.

2. Verfahren zur Herstellung flüssiger Aminaddukte nach Anspruch 1, dadurch gekennzeichnet, daß $R_F$ für einen Perfluoralkylrest mit 4 bis 8 Kohlenstoffatomen steht.

3. Verfahren zur Herstellung flüssiger Aminaddukte nach Anspruch 1 oder 2, dadurch gekennzeichnet, daß n gleich 0 ist.

4. Verfahren zur Herstellung flüssiger Aminaddukte nach einem oder mehreren der Ansprüche 1 bis 3, dadurch gekennzeichnet, daß vor der Filtration ein Filterhilfsmittel und/oder Kristallisationshilfsmittel zugesetzt werden.

5. Verfahren zur Herstellung flüssiger Aminaddukte nach Anspruch 4, dadurch gekennzeichnet, daß vor der Filtration Kieselsäurederivate und/oder Zellulosepulver zugesetzt werden.

6. Verfahren zur Herstellung flüssiger Aminaddukte nach einem oder mehreren der Ansprüche 1 bis 3, dadurch gekennzeichnet, daß vor der Filtration Wasser in einer Menge von 1 bis 4 Gew.-%, bezogen auf die Masse des Produktes, zugesetzt wird.

7. Verfahren zur Herstellung flüssiger Aminaddukte nach Anspruch 6, dadurch gekennzeichnet, daß Wasser in einer Menge von 1,5 bis 2,5 Gew.-%, bezogen auf die Masse des Produktes, zugesetzt wird.

**Claims**

1. Method for the preparation of liquid amine adducts of amides containing fluoride groups, of the general formula (I)

$$R_F\text{-}(CH_2)_n\text{-}SO_2\text{-}NHR_H \;\cdot\; N(R^1R^2R^3) \qquad\qquad (I),$$

wherein

$R_F$ is a perfluoroalkyl radical having 3 to 10 carbon atoms or a fluoroalkyl radical having 3 to 10 carbon atoms,

n represents an integer between 0 and 6,

$R_H$ represents a hydrogen atom or a linear alkyl radical having 1 to 4 carbon atoms,

$R^1$, $R^2$ and $R^3$ independently of one another represent a linear alkyl radical having 1 to 4 carbon atoms, characterised in that a sulphonic acid halide containing fluoride groups, of the general formula (II)

$$R_F(CH_2)_n\text{-}SO_2\text{-}X \qquad\qquad (II),$$

wherein

$R_F$ and n are as defined above and

X represents a halogen atom,

a tertiary amine $N(R^1R^2R^3)$ and ammonia or a primary amine $H_2NR_H$, wherein $R^1$, $R^2$, $R^3$ and $R_H$ are as defined above, are simultaneously reacted together without solvent, and the product obtained is freed from precipitated ammonium halide by filtration at a pressure of 1 to 6 bar and at a temperature of 20 to 120°C.

2. Method for the preparation of liquid amine adducts according to claim 1, characterised in that $R_F$ represents a perfluoroalkyl radical having 4 to 8 carbon atoms.

3. Method for the preparation of liquid amine adducts according to claim 1 or 2, characterised in that n equals 0.

4. Method for the preparation of liquid amine adducts according to one or more of claims 1 to 3, characterised in that a filter aid and/or a crystallisation auxiliary agent are added prior to filtration.

5. Method for the preparation of liquid amine adducts according to claim 4, characterised in that silica derivatives and/or cellulose powder are added prior to filtration.

6. Method for the preparation of liquid amine adducts according to one or more of claims 1 to 3, characterised in that prior to filtration water is added in a quantity of from 1 to 4 wt.%, referred to the mass of the product.

7. Method for the preparation of liquid amine adducts according to claim 6, characterised in that water is added in a quantity of from 1.5 to 2.5 wt.%, referred to the mass of the product.

**Revendications**

1. Procédé de préparation de produits d'addition liquides d'une amine avec un amide contenant des groupes fluorés de formule générale (I)

$$R_F\text{-}(CH_2)_n\text{-}SO_2\text{-}NHR_H \ \cdot \ N(R^1R^2R^3) \hspace{4cm} (I),$$

dans laquelle

$R_F$      représente un radical d'alkyle perfluoré ayant de 3 à 10 atomes de carbone ou un radical d'alkyle fluoré ayant de 3 à 10 atomes de carbone,

n      représente un nombre entier compris entre 0 et 6,

$R_H$      représente un atome d'hydrogène ou un radical alkyle linéaire ayant de 1 à 4 atomes de carbone,

$R^1$, $R^2$ et $R^3$      représentent, indépendamment les uns des autres, un radical alkyle linéaire ayant de 1 à 4 atomes de carbone.

caractérisé en ce que l'on fait réagir un halogénure d'acide sulfonique contenant des groupes fluorés de formule générale (II)

$$R_F(CH_2)_n\text{-}SO_2\text{-}X \hspace{5cm} (II),$$

dans laquelle $R_F$ et n ont les mêmes significations que ci-dessus, et

X      représente un atome d'halogène,

une amine tertiaire ($N(R^1, R^2, R^3)$) et de l'ammoniac ou une amine primaire $H_2NR_H$, ou $R^1$, $R^2$ et $R^3$ et $R_H$ ont les mêmes significations que ci-dessus simultanément et sans solvant et le produit obtenu est séparé par filtration sous une pression de 1 à 6 bar et à une température entre 20 et 120°C des halogénures d'ammonium précipités.

2. Procédé de préparation de produits d'addition liquides d'une amine selon la revendication 1, caractérisé en ce que $R_F$ représente un radical alkyle perfluoré ayant de 4 à 8 atomes de carbone.

3. Procédé de préparation de produits d'addition liquides d'une amine selon la revendication 1 ou 2, caractérisé en ce que n est égal à 0.

4. Procédé de préparation de produits d'addition liquides d'une amine selon une ou plusieurs des revendications 1 à 3, caractérisé en ce que l'on ajoute un adjuvant de filtration et/ou un adjuvant de cristallisation avant la filtration.

5. Procédé de préparation de produits d'addition liquides d'une amine selon la revendication 4, caractérisé en ce que l'on ajoute des dérivés de l'acide silicique et/ou de la cellulose en poudre avant la filtration.

6. Procédé de préparation de produits d'addition liquides d'une amine selon une ou plusieurs des revendications 1 à 3, caractérisé en ce que l'on ajoute , avant la filtration, de l'eau en quantité allant de 1 à 4% en poids rapportée à la masse du produit.

7. Procédé de préparation de produits d'addition liquides d'une amine selon la revendication 6, caractérisé en ce que l'on ajoute de l'eau en quantité allant de 1,5 à 2,5% en poids rapportée à la masse du produit.